(19) Europäisches Patentamt
European Patent Office
Office européen des brevets

(11) **EP 4 238 099 B1**

(12) **EUROPEAN PATENT SPECIFICATION**

(45) Date of publication and mention
of the grant of the patent:
**06.05.2026 Bulletin 2026/19**

(21) Application number: **21802602.9**

(22) Date of filing: **22.10.2021**

(51) International Patent Classification (IPC):
**G16B 50/40** *(2019.01)*

(52) Cooperative Patent Classification (CPC):
**G16B 50/40**

(86) International application number:
**PCT/EP2021/079305**

(87) International publication number:
**WO 2022/090067 (05.05.2022 Gazette 2022/18)**

(54) **METHOD OF ANONYMIZING GENOMIC DATA**

VERFAHREN ZUR ANONYMISIERUNG GENOMISCHER DATEN

MÉTHODE D'ANONYMISATION DES DONNÉES GÉNOMIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.10.2020 US 202063106921 P**

(43) Date of publication of application:
**06.09.2023 Bulletin 2023/36**

(73) Proprietor: **Koninklijke Philips N.V.**
**5656 AG Eindhoven (NL)**

(72) Inventors:
• **HULSEN, Tim**
**5656 AE Eindhoven (NL)**
• **PLETEA, Daniel**
**5656 AE Eindhoven (NL)**

(74) Representative: **Philips Intellectual Property & Standards**
**High Tech Campus 52**
**5656 AG Eindhoven (NL)**

(56) References cited:
**US-A1- 2016 125 141    US-A1- 2019 259 473**
**US-A1- 2020 035 332**

• **KALE GULCE ET AL: "A utility maximizing and privacy preserving approach for protecting kinship in genomic databases", BIOINFORMATICS, vol. 34, no. 2, 15 January 2018 (2018-01-15), GB, pages 181 - 189, XP055834251, ISSN: 1367-4803, Retrieved from the Internet <URL:https://academic.oup.com/bioinformatics/article-pdf/34/2/181/25114242/btx568.pdf> [retrieved on 20211206], DOI: 10.1093/bioinformatics/btx568**

• **HUMBERT MATHIAS ET AL: "De-anonymizing Genomic Databases Using Phenotypic Traits", PROCEEDINGS ON PRIVACY ENHANCING TECHNOLOGIES, vol. 2015, no. 2, 1 June 2015 (2015-06-01), pages 99 - 114, XP055869543, Retrieved from the Internet <URL:https://petsymposium.org/2015/papers/07_Humbert.pdf> [retrieved on 20211206], DOI: 10.1515/popets-2015-0020**

**Description**

**FIELD**

The presently disclosed subject matter relates to a method for anonymizing a genomic data set and a corresponding system for anonymizing a genomic data set. The presently disclosed subject matter further relates to a computer-readable medium.

**BACKGROUND**

Whole genome sequencing is getting cheaper and cheaper, and services like 23andMe and AncestryDNA offer to sequence hundreds of thousands of SNPs for prices around $100. However, as so much genomic information becomes available, concerns for privacy and security grow. Adversaries are increasingly able to combine genotypic and phenotypic information in a variety of ways to de-anonymize genomic databases. An identification attack, for example, is an attack in which the adversary attempts to identify the genotype (among multiple genotypes) that corresponds to a given phenotype. A further type of de-anonymization attack is the perfect matching attack, where the adversary attempts to match multiple phenotypes to their corresponding genotypes. Statistical models may also be used by an adversary to predict phenotypic traits, based on whole-genome sequencing data. Because of current advancements in genomics, the risk of identification of a subject using their genomic data is growing rapidly.

Quasi-identifiers, also known as indirect identifiers, are fields in a dataset that can be used in combination with one another to identify individuals. Examples include gender, zip code, birth date, profession and income. While there are many people who share the same gender, birth date or ZIP code, the combination of these for any one person may be unique, particularly if that person resides in a rural area with a small population. Examples of indirect identifiers include phenotypic traits, such as hair color and eye color, among many others.

Currently, whole genome sequences can be easily connected to phenotypic traits, making it possible to find out eye color, hair color, skin color, blood type, and the like, and subsequently identify the subject. As progress is made in genomic research, this problem will worsen. Often, users and researchers choose one of these two options: keep all genomic information intact, thereby risking a privacy breach, or remove all potentially identifiable information from the dataset, which limits the usefulness of the data.

Published US patent application US 2020/0035332 A1 describes methods and systems for anonymizing genetic data. The methods and systems described therein identify ancestry identification marker (AIM) regions in the genetic data. The AIM regions of the genetic data includes single nucleotide polymorphism (SNP) alleles associated with a population of patients belonging to a certain ancestry. AIM regions which do not contain gene variants associated with a specific disease may then be masked or removed from the genetic data.

A problem of the prior art is that there is no guarantee that the resulting genetic data is sufficiently anonymized. Merely masking or removing AIM regions without clinically relevant data may, in some cases, still result in a genetic data set which can re-identify the person. Moreover, the approach of the prior art involves removing data which may contribute in some as-yet unknown way to a particular disease, meaning that there is a possibility that useful information may be lost.

Removing more data from the genetic data set increases the risk of losing valuable and relevant information, thereby reducing the usefulness of the data, but preserving more data in the genetic data set increases the risk of the individual being re-identified from their genetic data set. There is therefore an advantage to being able to ensure that the genetic data set is sufficiently anonymized whilst preserving as much information as possible for applications such as research. Quantifying a risk of re-identification and ensuring that the risk that a person can be re-identified from an anonymized genomic data set can therefore improve patient privacy, security, and the amount of information available to researchers in an anonymized genomic data set.

Kale Gulce ET AL: "A utility maximizing and privacy preserving approach for protecting kinship in genomic databases", Bioinformatics vol 34, no 2, 2018 teaches how one can use a kinship metric to remove some genomic information of individuals added to a large genomics data set if family members (having similar genomes) are already present in the database.

Humbert Mathias et al: "De-anonymizing Genomic Databases Using Phenotypic Traits", Proceedings on Privacy Enhancing Technologies, vol. 2015, no. 2, 1 June 2015 teaches re-identification attacks of genomic information based on externally visible phenotypic traits, the information of which can be collected from other data sources such as social networks.

SUMMARY

It would be advantageous to preserve as much genomic data as possible for researchers to access, whilst also protecting the privacy and security of the individuals whose data is used. A system and computer-implemented method for anonymizing a genomic data set are set out herein and are claimed. Said system and computer-implemented method aim to address these and other concerns.

Existing methods for genomic data preparation either remove important research information from the genomic data set, for example by removing all genomic data relating to visible phenotypic traits regardless of whether said genomic data also relates to a disease of interest, thereby reducing the amount of knowledge that can be gained from its analysis, or preserve too much identifying information of the individual, risking security and privacy breaches.

The presently disclosed subject matter includes a computer-implemented method for anonymizing a genomic data set, a system for anonymizing a genomic data set and a computer-readable medium. The method for anonymizing a genomic data set may comprise receiving the genomic data set. The genomic data set may comprise a plurality of alleles arranged in a plurality of single nucleotide polymorphisms (SNPs), the plurality of SNPs comprising one or more phenotype informative SNPs. A phenotype informative SNP may be an SNP which relates to a phenotypic trait. The genomic data set may correspond to a genome of a person. The method may further comprise obtaining a phenotypic probability for at least one phenotype informative SNP. The phenotypic probability may be a probability of the phenotypic trait being expressed as a result of the at least one allele corresponding to the at least one phenotype informative SNP. For example, if the phenotypic trait is "blue eyes", the phenotypic probability may be the probability that the alleles occupying a particular phenotype informative SNP associated with eye color will result in the presentation of blue eyes. The method may further comprise obtaining a proportion of a population which exhibits said phenotypic trait. For example, if the phenotypic trait is "blue eyes", the proportion of the population would correspond to the proportion of the population having blue eyes. The method further comprises computing a re-identification risk score based on the genomic data set. The re-identification risk score indicates a risk of re-identifying the person associated with the genomic data set from the genomic data set. The re-identification risk score may be computed from the obtained phenotypic probability and the obtained proportion of the population which exhibits said phenotypic trait. The re-identification risk score may then be compared to a threshold risk criterion. If the re-identification risk score does not meet the threshold risk criterion, the method may comprise anonymizing the genomic data set by selecting a phenotype informative SNP and masking the selected phenotype informative SNP. If the re-identification risk score meets the threshold risk criterion, the method may comprise outputting the anonymized genomic data set.

Embodiments help to improve the privacy and security associated with genomic data whilst also improving the amount of information available, for example to researchers. Various examples and embodiments are provided herein describing how the re-identification risk score is determined and how the genomic data set is anonymized.

By using a threshold risk criterion, the level of acceptable risk may be taken into account and a genomic data set may be accordingly anonymized. Moreover, the amount of information retained in the genomic data set may be maximized by avoiding removing clinically relevant unnecessarily.

Aspects of the presently disclosed subject matter include a corresponding system for anonymizing a genomic data set.

Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

Another aspect of the presently disclosed subject matter provides a method of making the computer program available for downloading. This aspect is used when the computer program is uploaded into, e.g., Apple's App Store, Google's Play Store, or Microsoft's Windows Store, and when the computer program is available for downloading from such a store.

BRIEF DESCRIPTION OF THE DRAWINGS

Further details, aspects, and embodiments will be described, by way of example, with reference to the drawings. Elements in the figures are illustrated for simplicity and clarity and have not necessarily been drawn to scale. In the Figures, elements which correspond to elements already described may have the same reference numerals. In the drawings,

Figure 1 schematically shows an example of an embodiment of a system for anonymizing a genomic data set,
Figure 2 schematically shows an example of a genomic data set,
Figure 3 schematically shows an example of an embodiment of a method for calculating a re-identification risk score,
Figure 4 schematically shows an example of an embodiment of a method for anonymizing a genomic data set,
Figure 5 schematically shows an example of a computer readable medium having a writable part comprising a computer program according to an embodiment, and

Figure 6 schematically shows a representation of a processor system according to an embodiment.

List of Reference Numerals:

| | |
|---|---|
| 100 | a system |
| 110 | a processor subsystem |
| 120 | an external network |
| 130 | an input/output subsystem |
| 140 | a memory |
| 142 | a genomic data set |
| 144 | instructions |
| 150 | a data interface |
| 200 | a genomic data set |
| 330 | a database |
| 1000 | a computer readable medium |
| 1010 | a writable part |
| 1020 | a computer program |
| 1110 | integrated circuit(s) |
| 1120 | a processing unit |
| 1122 | a memory |
| 1124 | a dedicated integrated circuit |
| 1126 | a communication element |
| 1130 | an interconnect |
| 1140 | a processor system |
| 1100 | a device |

## DETAILED DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the presently disclosed subject matter is not limited to the embodiments, as features described herein or recited in mutually different dependent claims may be combined.

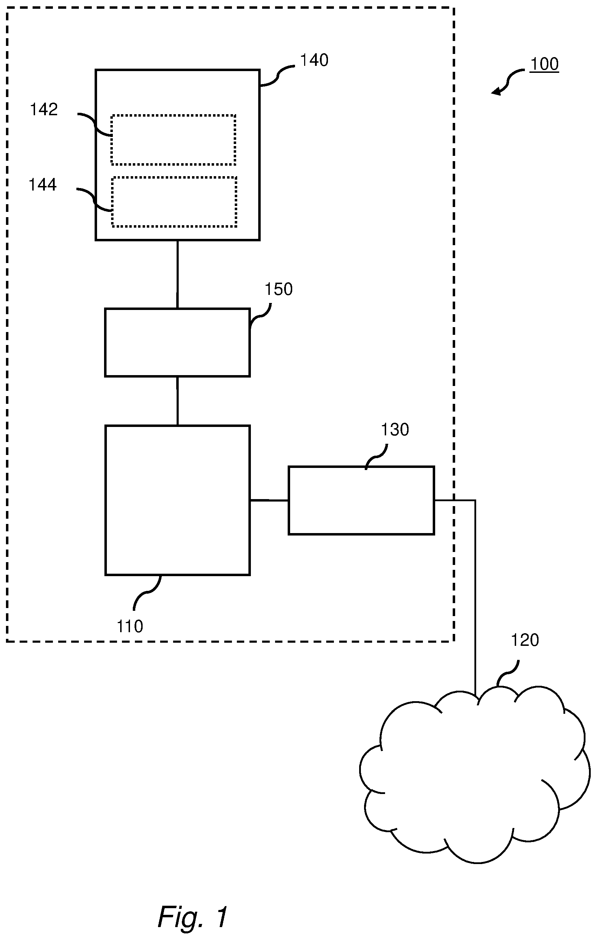

**Figure 1** schematically shows an example of an embodiment of a system 100 for anonymizing a genomic data set. System 100 may comprise a processor subsystem 110 and an input/output subsystem 130. In some embodiments, system 100 may further comprise a memory 140, which may be accessed via a data interface 150. Memory 140 may be a local memory, or may be a remote memory. In some embodiments, system 100 may be communicatively coupled to an external network or external entity 120.

In an embodiment, input/output (IO) subsystem 130 may comprise an interface for receiving input and/or outputting an output. For example, the IO subsystem 130 may be configured to receive a genomic data set corresponding to a genome of a person.

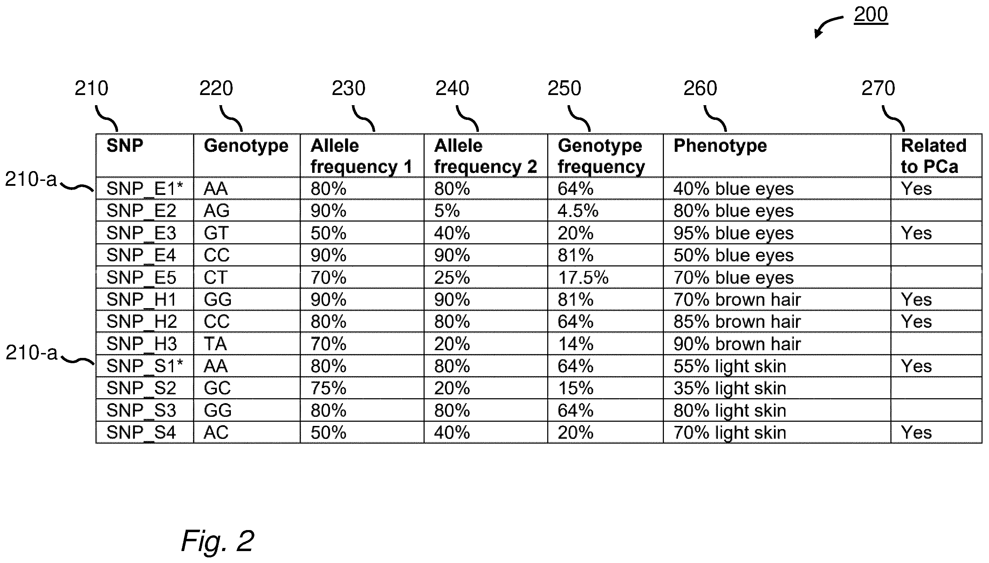

The genomic data set may comprise a plurality of alleles arranged in a plurality of single nucleotide polymorphisms (SNPs). A SNP indicates a position in the genome at which gene variations typically occur, and each allele is a variant form of a given gene, genetic sequence or SNP. That is, a SNP indicates a single genomic position at which at least a proportion of the population have different nucleotides at that position. SNP data may therefore be considered mutation data, and said mutation data can be used to identify the person from the genomic data set. Most commonly, a SNP corresponds to a pair of alleles, which may be nucleobases (adenine (A), cytosine (C), thymine (T) or guanine (G)). In autosomes, for example, one allele is inherited from the mother and one allele is inherited from the father. For each SNP, it is typically known what the wild type allele is and what the mutant allele is. A wild type allele is the allele which typically produces the phenotype most frequently found in a population, whereas the mutant allele is an allele which produces a phenotype other than the wild type phenotype. The alleles occupying the SNPs may be referred to as the genotype. The alleles making up each SNP may have an associated genotypic frequency, which may indicate how frequently said alleles occur in a population (e.g. the population of a region, a country, a continent, the world, a dataset, etc.) at the position of said SNP, and an associated phenotypic probability, which indicates the probability of said alleles producing a particular phenotypic trait. Many SNPs may contribute, or correspond to, one or more phenotypic traits. Such SNPs may be referred to as phenotype informative SNPs. Examples of phenotypic traits include exterior phenotypic traits, such as eye color, skin color, hair color or the like, and/or interior phenotypic traits, such as blood type, predisposition to diseases, lactose intolerance and the like. Such phenotypic traits may be considered to be indirect identifiers, as although such a trait may not on its own identify an individual, combinations of many such traits reduce the number of potential people to which the genome corresponding to the genomic data set may belong, and may ultimately identify a particular individual. In some embodiments, the genomic data set may comprise demographic data, such as age information, address information or the like. A simulated example snippet of a genomic data set is provided in Figure 2 and will be elucidated further in the corresponding description thereof.

In an embodiment, the IO subsystem 130 may be configured to receive an indication of a disease to be studied. A user, for example a researcher, may be interested in studying or researching a particular disease. Said disease may be known to correspond to a selection of SNPs. For example, a user may be interested in researching prostate cancer. The IO subsystem 130 may receive a user input indicating an interest in prostate cancer, and may provide, to the user or to another subsystem or process of the system 100, a list of SNPs that are known to relate or contribute to prostate cancer. In some embodiments, a user may select or indicate a particular disease via the IO subsystem 130, and the known related SNPs may be retrieved, for example from an external source or from an internal memory such as memory 140. In some embodiments, the disease of interest may be predetermined or indicated in, or obtained from, the genomic data set. In some cases, SNPs that are known to contribute to a particular disease may also contribute to phenotypic traits, such as eye color or blood type.

The IO subsystem 130 may be further configured to store the received genomic data set in memory 140. The IO subsystem 130 may, in some embodiments, be configured to receive user input, such as an indication of a particular disease to be studied, or a selection of data within the genomic data set to be prioritized. The IO subsystem 130 may, in some embodiments, be configured to receive a target re-identification risk score to indicate a desired level of anonymization. For example, the target re-identification risk score may be used as a threshold risk criterion.

In some embodiments, the IO subsystem 130 may be configured to access an external network 120. External network 120 may comprise a cloud-based network, a server, an external database, an external device or the like. In some embodiments, the genomic data set(s), the threshold risk criterion and/or information on at least one disease may be stored in the external network 120 and accessed via the IO subsystem 130.

The IO subsystem 130 may, in some embodiments, comprise an input device configured to receive an input from a user, such as a touchscreen, a keyboard, a mouse, a trackpad or the like, or a sensor input, such as a camera, microphone, proximity sensor or the like. The IO subsystem 130 may, in some embodiments, comprise an output device such as a display, a speaker or the like, to provide an output to a user. In some embodiments, the IO subsystem 130 may be configured to process inputs and/or outputs from/to additional components, subsystems, or external entities. For example, the IO subsystem 130 may be configured to receive an input from an external device, a network such as a cloud-based network, a server, or a component of the system 100.

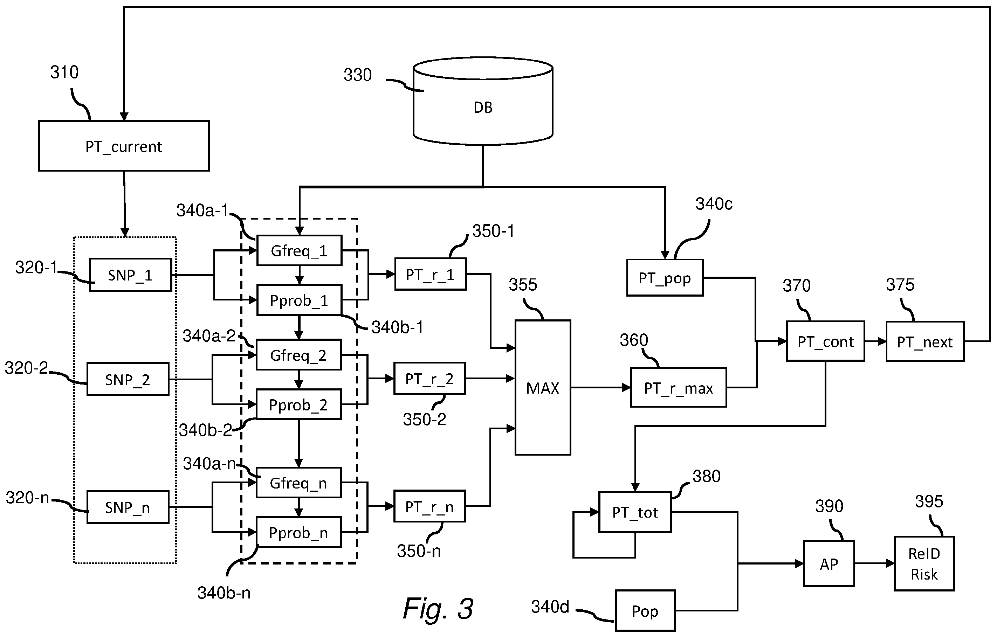

In an embodiment, memory 140 may be configured to store one or more genomic data sets, for example in a database for storing genomic data sets of a plurality of people. Additionally or alternatively, memory 140 may be configured to store instructions or information for use in the method for anonymizing a genomic data set. Memory 140 may also store a threshold risk criterion. The threshold risk criterion may be a criterion used to ensure that the anonymized genomic data set is sufficiently anonymized prior to being output or distributed, for example by ensuring that the re-identification risk score of the genomic data set complies with a specified risk level. The calculation of a re-identification risk score will be described in detail with reference to Figure 3. The threshold risk criterion may be, for example, a percentage, indicating a risk of re-identifying the person from the genomic data set, or an applicable population size, indicating the number of people having said phenotypic traits. A large number of people sharing the phenotypic traits corresponds to a low risk of re-identification. In some cases, such as when the starting population is small, use of a threshold applicable population size as the re-identification risk criterion may be particularly suitable.

Memory 140 may comprise at least one database, such as genomic database 142 and/or SNP database 144. Genomic database 142 may be configured to store one or more genomic data sets corresponding to a respective one or more people. SNP database 144 may be configured to store SNP information relating to one or more diseases, such as lists of SNPs corresponding to a particular disease. The memory 140 may be implemented as an electronic memory, for example a flash memory, or magnetic memory, say hard disk or the like, or optical memory, e.g., a DVD. The memory 140 may comprise multiple discrete memories together making up the memory 140. The memory 140 may comprise a temporary memory, e.g. a RAM. In the case of a temporary memory 140, memory 140 may be associated with a retrieving device to obtain data before use and to store the data in the storage, say by obtaining them over an optional network connection (not shown).

In an embodiment, the memory 140 may comprise a local memory and/or an external (e.g. remote) memory. For example, the genomic database 142 may be stored in a local memory. The SNP database 144 may be stored externally and, in some cases, may be merely accessed by the system 100. In another example, the genomic database 142 may be stored externally, such as in a central (e.g. government) database. The genomic database 142 and the SNP database 144 may be stored in the same storage location, or in different storage locations.

The processor subsystem 110 may comprise at least one processor, and may be referred to as at least one processor circuit. In an embodiment, processor subsystem 110 may be configured to determine a re-identification risk score of a genomic data set and to anonymize the genomic data set. In some embodiments, the processor subsystem 110 may be configured to preprocess the genomic data set, for example by masking, e.g. deleting, any direct identifiers therein. A direct identifier may be a SNP whose data independently identifies an individual without the need for additional information, such as data relating to other SNPs. In some embodiments, the processor subsystem 110 may preprocess the genomic data set by obtaining a list of SNPs of interest to the user, for example by obtaining a list of SNPs that relate to or contribute to a particular specified disease from the user directly or from a database in memory 140 or via an external network 120. In some embodiments, the processor subsystem 110 may be configured to mask, e.g. delete, SNPs of the genomic data set that do not relate to the specified disease, for example by masking, e.g. deleting, SNPs that are not included in the obtained list of relevant SNPs. In some embodiments, knowledge of the SNPs contributing or relating to a particular disease is incomplete, and a researcher may prefer not to limit the genomic data set based on an incomplete list of SNPs.

The processor subsystem 110 may be configured to calculate a re-identification risk score from the genomic data set. The calculation of the re-identification risk score may be based on one or more phenotypic traits. For each specified phenotypic trait, the genotypic frequency of one or more phenotype informative SNPs relating to said phenotypic trait, the phenotypic probability of said phenotype informative SNPs producing said phenotypic trait, and a proportion of a population which has said phenotypic trait may be used in the calculation of the re-identification risk score. These terms will be more fully described with reference to Figure 2, and the calculation of the re-identification risk score will be described in detail with reference to Figure 3.

The processor subsystem 110 may be configured to compare the calculated re-identification risk score to a threshold risk criterion. The threshold risk criterion, which may also be referred to as a threshold re-identification risk criterion, may be stored locally, such as in memory 140, received from a user as user input via IO subsystem 130, or obtained from an external network 120 via IO subsystem 130, for example. If calculated re-identification risk score meets the threshold risk criterion, then the genomic data set is sufficiently anonymized and may be output, for example to an external device or to a user, or stored in, e.g. local, memory. If the calculated re-identification risk score does not meet the threshold risk criterion, then the genomic data set is not yet sufficiently anonymized, and the processor subsystem 110 may be configured to anonymize the genomic data set by masking, e.g. deleting, data corresponding to one or more SNPs.

The system 100 may further comprise a data interface 150. The data interface 150 may comprise a connector, e.g., a wired connector, e.g., an Ethernet connector, an optical connector, etc., or a wireless connector, e.g., an antenna, e.g., a Wi-Fi, 4G or 5G antenna. The data interface 150 may provide access to a memory 140.

The various subsystems of the system 100 may be disposed within a single device, or may communicate with each other over a computer network. The computer network may be an internet, an intranet, a LAN, a WLAN, etc. The computer network may be the Internet. The computer network may be wholly or partly wired, and/or wholly or partly wireless. For example, the computer network may comprise Ethernet connections. For example, the computer network may comprise wireless connections, such as Wi-Fi, ZigBee, and the like. The subsystems may comprise a connection interface which is arranged to communicate with other subsystems of system 100 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna. The computer network may comprise additional elements, e.g., a router, a hub, etc.

**Figure 2** schematically shows an example of a genomic data set 200. The genomic data set 200 may comprise a plurality of parameters for each SNP, such as those depicted in Figure 2 - SNP 210, which indicates a position or other identifier of a SNP, Genotype 220, which indicates the alleles at said SNP, Allele frequency 1 230, which indicates the frequency of the first allele in the plurality of alleles occupying said SNP, Allele frequency 2 240, which indicates the frequency of the second allele in the plurality of alleles occupying said SNP, Genotype frequency 250, which indicates the frequency of the genotype, e.g. the alleles of the SNP, occurring in a population such as a regional or global population, or in some cases a population of a dataset, Phenotype 260 (also referred to as phenotype probability 260), which indicates the probability of a particular phenotypic trait being produced by the genotype of said SNP and Disease Correlation 270, which indicates whether a SNP has a known correlation to a particular disease of interest. It is to be understood that a genomic data set may not comprise all of the listed parameters and the genomic data set may comprise parameters other than those listed, and the listed parameters are merely illustrative. For example, a genomic data set may comprise merely SNP 210 and Genotype 220, and any further information such as genotype frequency, phenotype probability and/or disease correlation may be obtained by querying a database or accessing a data source, e.g. via the external network 120. For example, based on an entry for SNP 210-a (SNP_E1) and the associated genotype ("AA" in the table of Figure 2), the system 100 may be configured to look up an associated genotype frequency, which, in this example, is the frequency of genotype AA occupying SNP_E1, in a population (64% in this simulated example), a phenotype probability - in this example, the probability that the genotype AA at SNP_E1 will produce blue eyes is 40%, and whether or not SNP_E1 is known to have any relevance to prostate cancer (prostate cancer is denoted as PCa in the table of Figure 2). For example, genotype frequency, phenotype probability and any relevant parameters may be obtained or accessed from the same data source, such as a single database, or from various data sources.

The sample of simulated genomic data set 200 shown in Figure 2 includes data corresponding to a plurality of phenotype informative SNPs. The genomic data set 200 corresponds to the simulated genome of a person having blue eyes, brown hair and light skin. In this example, it is assumed for the sake of simplicity that these three phenotypes form a set of indirect identifiers based on which a re-identification risk score is calculated. However, the use of these phenotypic traits is merely illustrative, and are non-limiting. More or fewer phenotypic traits may be used. This example, in which these three phenotypic traits are considered, will be followed throughout the present disclosure, to illustrate the methods and devices described herein.

In this example, the first phenotypic trait is "blue eyes". SNPs which contribute to or affect eye color may be SNP_E1, SNP_E2, SNP_E3, SNP_E4 and SNP_E5. According to the genomic data set of a particular individual, SNP_E1 is populated by genotype AA (e.g., the genotype comprises two alleles, each of which are adenine nucleotides). The frequency of genotype AA at the position in the genome corresponding to SNP_E1 in a population is 64% (according to this simulated example). The probability of this genotype AA at this position resulting in blue eyes is 40%, and SNP_E1 is known to contribute or relate to prostate cancer.

Similarly:

- SNP_E2 is populated by genotype AG, which has a genotype frequency of 4.5% and is 80% likely to result in blue eyes, and has no known correlation or contribution to prostate cancer;
- SNP_E3 is populated by genotype GT, with genotype frequency of 20%, with a 95% probability of producing blue eyes, and has a known correlation to prostate cancer;
- SNP_E4 is populated by genotype CC, with a genotype frequency of 81% and a 50% probability of producing blue eyes, with no known correlation to prostate cancer; and
- SNP_E5 is populated by genotype CT, with a genotype frequency of 17.5% and a 70% probability of producing blue eyes, with no known correlation to prostate cancer.

Continuing with this example, SNP_H1, SNP_H2 and SNP_H3 are SNPs relating to hair color, and SNP_S1, SNP_S2, SNP_S3 and SNP_S4 are SNPs relating to skin color. Of particular note is that SNP_E1 and SNP_H1, each denoted by 210-a, are the same SNP - that is, they correspond to the same position in the genome and are populated by the same alleles. This particular SNP contributes to both eye color and skin color, and the genotype AA populating said SNP has a 40% probability of producing blue eyes and a 55% probability of producing light skin.

The re-identification risk score may be calculated based on parameters such as those shown in the table of Figure 2. This calculation will be described in further detail with reference to Figure 3.

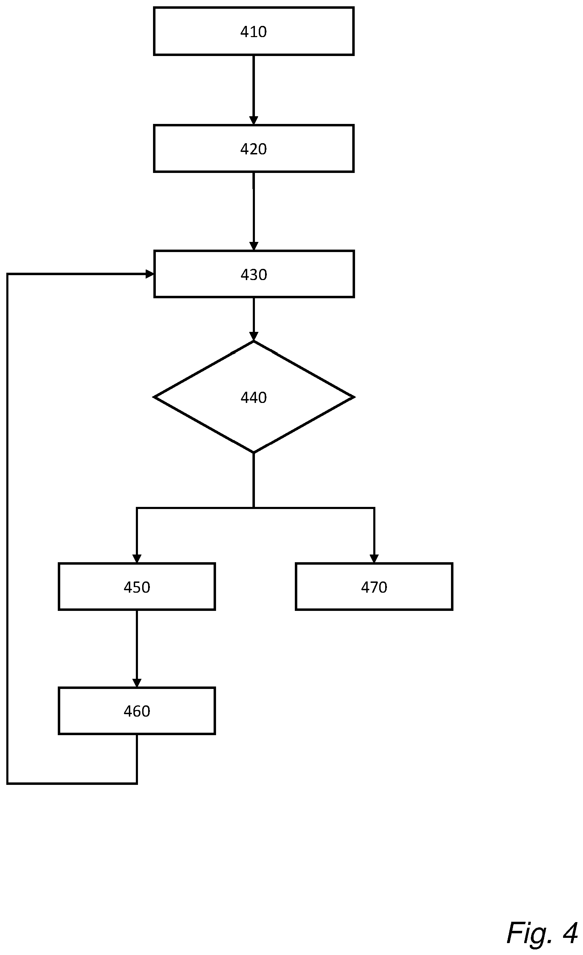

Once calculated, the re-identification risk score may be compared to a threshold risk criterion, which may be an applicable population or a percentage, for example. The applicable population may correspond to a proportion of the population of a region (e.g. country, world etc.) or the population of the dataset, or the like. Further details regarding the threshold risk criterion will be further described with reference to Figure 4. If the re-identification risk score does not meet the threshold risk criterion, then data corresponding to one or more phenotype informative SNPs may be masked and the re-identification risk score may be re-calculated, without the data of the one or more masked phenotype informative SNPs. Masking an SNP may comprise deleting the data corresponding to the SNP, replacing the data corresponding to the SNP with null data, or any known masking method, for example. The process of masking one or more SNPs and recalculating the re-identification risk score may be repeated until the re-identification risk score meets the threshold risk criterion.

**Figure 3** schematically shows an example of an embodiment of a method for calculating a re-identification risk score. A first phenotypic trait, **PT_current** 310 may be selected. The first, or current, phenotypic trait **PT_current** 310 may be selected from a list of phenotypic traits to be considered in the calculation of the re-identification risk score, or from a list of all known phenotypic traits. In some embodiments, the list of phenotypic traits from which first phenotypic trait **PT_current** 310 may be selected may comprise exterior phenotypic traits, such as eye color, hair color and the like, interior phenotypic traits, such as blood type and the like, or a combination of both exterior phenotypic traits and interior phenotypic traits. In some embodiments, the list of phenotypic traits to be considered in the calculation of the re-identification risk score may be received as an input from a user or from another subsystem or device. In some embodiments, the list of phenotypic traits to be considered may be obtained from a database or may be determined based on the phenotypic traits exhibited by the individual whose genomic data set is to be anonymized. For example, if the person has blue eyes, brown hair and light skin, these phenotypic traits may be included in the list of phenotypic traits to be considered in the calculation of re-identification risk score.

The first phenotypic trait **PT_current** 310 may be correlated to one or more phenotype informative SNPs present in the genomic data set. That is, one or more positions on the genome may be known to contain mutations which result, or contribute to, the expression of the first phenotypic trait. For the sake of illustration, these SNPs are shown as **SNP_1** 320-1, **SNP_2** 320-2 and **SNP-n** 320-n, although it is to be understood that there may be more or fewer SNPs for a particular phenotypic trait, and that the same SNP may contribute to multiple phenotypic traits, to the same or different extents.

For at least one of the identified SNPs, taking **SNP_1** 320-1 as an example, a genotypic frequency **Gfreq_1** 340a-1 and a phenotypic probability **Pprob_1** 340b-1 may be obtained, for example from a database **DB** 330. Database **DB** 330 may be stored in a local memory such as memory 140 or in an external memory, such as in cloud storage or in an external device. For example, Database **DB** 330 may be accessed via an external network such as external network 120. In some embodiments, database **DB** 330 may be a central database to be accessed by researchers from an organization, collaboration or the like. In some embodiments, the genomic data set may comprise one or more of the genotypic frequency **Gfreq_1** 340a-1 and the phenotypic probability **Pprob_1** 340b-1, so that these values may be obtained without using a separate database. The genotypic frequency **Gfreq_1** 340a-1 may indicate a frequency of **SNP_1** 320-1 being occupied by the alleles indicated in the genomic data set at **SNP_1** 320-1. The phenotypic probability **Pprob_1** 340b-1 may indicate that a probability of those alleles producing, or resulting in, the first phenotypic trait. In some embodiments, the genotypic frequency and phenotypic probability may be combined to obtain a risk term for each SNP, for use in the calculation of a re-identification risk score. The risk term may be an intermediate value. For example, risk term **PT_r_1** 350-1 may be determined for **SNP_1** 320-1 by combining **Gfreq_1** 340a-1 and **Pprob_1** 340b-1. The risk term **PT_r_1** 350-1 may be, or may comprise, the product of the genotypic frequency **Gfreq_1** 340a-1 and the phenotypic probability **Pprob_1 340b-1,** or a sum of logarithmic values of **Gfreq_1** 340a-1 and **Pprob_1** 340b-1, or the like.

In some embodiments, these values may be obtained for each phenotype informative SNP - for example, genotypic frequency **Gfreq_2** 340a-2 and phenotypic probability **Pprob_2** 340b-2 of **SNP_2** 320-2, and genotypic frequency **Gfreq_n** 340a-n and phenotypic probability **Pprob**_n 340b-n of **SNP_n** 320-n. A risk term associated with each SNP may be calculated therefrom, for example to obtain a risk term **PT_r_2** 350-2 corresponding to **SNP_2** 320-2 and a risk term **PT_r_n** 350-n corresponding to **SNP_n** 320-n and so on.

In some embodiments, the largest risk term **PT_r_max** 360 may be determined, depicted by **MAX** 355. **MAX** 355 may return the largest risk term **PT_r_max** 360 of the risk terms **PT_r_1** 350-1 to **PT_r_n** 350-n corresponding to the SNPs **SNP_1** 320-1 to **SNP_n** 320-n. In some embodiments, **MAX** 355 may also return the SNP corresponding to the largest risk term **PT_r_max** 360. If, for example, the first phenotypic trait has three related SNPs - **SNP_1** 320-1, **SNP_2** 320-2 and **SNP-n,** 320-n - then **PT_r_max** 360 would be the largest **of PT_r_1** 350-1, **PT_r_2** 350-2 and **PT_r_n** 350-n. Although the above refers to the use of a maximum, it is to be understood that the present method is not limited thereto. For example, in some embodiments, the average risk term (e.g. the average risk term across SNPs 320 relating to a particular phenotypic trait) may be determined instead of the largest risk term. For example, the choice between using an average risk term and a maximum term may be based at least in part on the type of risk or attacker that the de-identification efforts are addressing.

In some embodiments, a proportion of the population **PT_pop** 340c which exhibits the first phenotypic trait may be obtained, for example from a database such as **DB** 330. The proportion of the population exhibiting the first phenotypic trait **PT_pop** 340c may be combined with the largest risk term **PT_r_max** 360, to obtain a contribution term corresponding to the first phenotypic trait, **PT_cont** 370. For example, the contribution term **PT_cont** 370 may be a quotient of **PT_pop** 340c and **PT_r_max** 360, or a difference between logarithmic terms of **PT_pop** 340c and **PT_r_max** 360. Once the contribution term **PT_cont** 370 has been determined, the method may comprise selecting a next phenotypic trait **PT_next** 375 and repeating the process, by setting **PT_next** 375 as **PT_current** 310, as indicated by the arrows in the flowchart of Figure 3.

The contribution term **PT_cont** 370 may also be combined with other contribution terms, for example contribution terms corresponding to other phenotypical traits, to obtain a total phenotypic trait term **PT_tot** 380. In the first iteration, e.g. for the first phenotypic trait, the total phenotypic trait term **PT_tot** 380 may be merely set to be the contribution term **PT_cont** 370 corresponding to the first phenotypic trait. In some embodiments, the total phenotypic trait term **PT_tot** 380 may be updated as the contribution term for each phenotypic trait is determined. For example, phenotypic trait contribution PT_cont 370 of the first phenotypic trait and contribution terms of other phenotypic traits may be multiplied, or added logarithmically, e.g. the logarithms of the contribution terms may be added. In some embodiments, contribution terms for each phenotypic trait of interest may be determined as described herein and combined once all of said contribution terms have been calculated, for example by finding the product of said contribution terms, or by finding the sum of the logarithms of said contribution terms.

In some embodiments, the total phenotypic trait term **PT_tot** 380 may be combined with a population size **Pop** 340d, which may be a regional or global population, or a proportion of a population that has been already determined, to obtain an applicable population **AP** 390. For example, if the user has an interest in researching prostate cancer in patients aged between 50 and 75, the population may be the number of people in the region of interest (e.g. in Europe, or in the United States, or globally, etc.) who are men between the ages of 50 and 75. The population size **Pop** 340d may be obtained from a database such as database **DB** 330, as an input from a user, from memory, or the like. For example, the applicable population **AP** 390 may be determined by multiplying the population size **Pop** 340d with the total phenotypic trait term **PT_tot** 380, or by equivalently summing the logarithms of the population size **Pop** 340d and the total phenotypic trait term **PT_tot** 380. The applicable population **AP** 390 may indicate the number of people whose genomes would be consistent with that of the genomic data set.

In some embodiments, the applicable population 390 may be used as the re-identification risk score **ReID Risk** 395, for example if the threshold risk criterion indicates a number of people. In some embodiments, the re-identification risk score **ReID Risk** 395 may be determined from the applicable population. For example, re-identification risk score **ReID Risk** 395 may be a risk that a particular individual may be identified from the genomic data set, and may be calculated as the inverse of the applicable population **AP** 390, e.g. 1/AP. This may be suitable if the threshold risk criterion is based on a risk level, for example determined or prescribed by ethical or privacy requirements.

An equation for calculating the re-identification risk score according to one embodiment is provided below:

$$AP = Pop * \prod_{i \in II} \frac{PT\_Pop_i}{\max_{s \in SNP_i} (Gfreq_{s,i} * Pprob_{s,i})}$$

(Equation 1)

$$ReID\ Risk = \frac{1}{AP}$$

(Equation 2)

Where:

- $i \in II$ indicates a phenotypic trait of a set *II* of phenotypic traits
- $s \in SNP_i$ indicates a SNP relating to phenotypic trait i
- $Gfreq_{s,i}$ indicates a genotypic frequency of the alleles populating the SNP s
- $Pprob_{s,i}$ indicates a phenotypic probability that the alleles populating the SNP s will result in phenotypic trait i.
- $\max_{s \in SNP_i}$ indicates the SNP of the set of SNPs corresponding to phenotypic trait i for which the product of $Gfreq_{s,i}$ and $Pprob_{s,i}$ is at a maximum
- Pop indicates a population size
- $PT\_Pop_i$ indicates a proportion of a population exhibiting phenotypic trait i

As indicated above, the calculation of the applicable population is not limited to the use of a maximum term ($\max_{s \in SNP_i}$). In some embodiments, for example, the maximum term $\max_{s \in SNP_i}$ may be replaced with an average term across the SNPs of the set of SNPs corresponding to the phenotypic trait i or the like.

Moreover, although the re-identification risk score is denoted here as simply being the inverse of the applicable population, it is to be understood that the calculation is not limited thereto. For example, additional dimensions may be used in the computation of the risk score in addition to the applicable population. Such dimensions may include, for example, one or more of: the power of the attacker (e.g. their ability to access various identification databases), the probability, or chance, of an attack (e.g. internal/external) based on existing context and thresholds and/or weights corresponding to the phenotypes and the population (e.g. the proportion of data subjects that have a re-identification risk higher than that threshold, dependencies between phenotypes that divide the applicable population and the like).

In some embodiments, an additional correction factor may be used to take into account dependencies between multiple phenotypic traits. The additional correction factor may be based at least in part on available statistics, such as those indicating an association between two traits or conditions. For example, consider the proportion of the population exhibiting a phenotypic trait of high BMI (body mass index) and heart disease. In general, obese people (obese being defined medically according to a BMI score exceeding a threshold) make up approximately 20% of the general population. However, obese people make up 40% of the population of people with heart disease. In this example, it is apparent that there is a relation between the phenotypes of high BMI and heart disease, and this relation can be used as the correction factor. For example, the correction factor may be to use a factor of 40% instead of a factor of 20% in calculating the proportion of the population used in the term **PT_Pop.**

**Figure 4** schematically shows an example of an embodiment of a computer-implemented method for anonymizing a genomic data set.

The method may comprise, in an operation entitled "RECEIVE GENOMIC DATA SET", receiving 410 a genomic data set, for example from a user or from a data source such as memory 150 or from an external source, e.g. via external network 120. In some embodiments, the genomic data set may be obtained after data corresponding to direct identifiers have been removed. That is, in some embodiments, the genomic data set may comprise data corresponding to indirect identifiers.

The method may comprise, in an operation entitled "OBTAIN PARAMETERS", obtaining 420, for at least one phenotypic trait, a population proportion, e.g. **PT_pop,** indicating a proportion of a population exhibiting said phenotypic trait, and for at least one phenotypic SNP corresponding to said phenotypic trait, a genotypic frequency, e.g. **Gfreq,** and a phenotypic probability, e.g. **Pprob,** as described with reference to Figure 3. In some embodiments, obtaining 220 parameters may comprise obtaining a population size, such as **Pop** 340d. In some embodiments, obtaining 420 parameters may comprise obtaining a list of phenotypic traits and/or a list of SNPs relating to each of at least one phenotypic trait.

The method may comprise, in an operation entitled "COMPUTE RE-IDENTIFICATION RISK SCORE", computing 430 a re-identification risk score for the genomic data set. Computing 430 the re-identification risk score may be performed, for example, as described with reference to Figure 3.

The method may comprise, in an operation entitled "COMPARE TO THRESHOLD", comparing 440 the computed re-identification risk score to a threshold risk criterion. If the re-identification risk score meets the threshold risk criterion, the method may proceed to an operation entitled "OUTPUT ANONYMIZED DATA SET", in which the genomic data set is output 470, for example to a user or to another subsystem, function or device. In some embodiments, outputting the anonymized data set may comprise storing the anonymized data set, for example in memory 140. In some embodiments, the genomic data set may be output to a database such as database **DB** 330, or the genomic data set may be output to an external device, e.g. via external network 120, such as to a central database or central storage device, in the cloud or in a remote device. In some embodiments, the genomic data set may be encrypted prior to outputting, e.g. storing or transmitting, the genomic data set. In some embodiments, the re-identification risk score may be output along with the anonymized data set. Outputting the re-identification risk score as well as the anonymized genomic data set may enable the anonymized data set to be used, e.g. in subsequent research or applications, which may have a different level of acceptable risk of re-identification (e.g. the threshold re-identification risk threshold may vary). By including the re-identification risk score of an anonymized genomic data set, if the re-identification risk score already meets the threshold re-identification risk threshold of the subsequent research or application, then re-anonymization may be avoided or at least reduced.

In some embodiments, the re-identification risk score may be computed as a percentage, for example by taking the inverse of the applicable population as shown in Equation 2. In such embodiments, the threshold risk criterion may take the form of a percentage indicating a risk of re-identifying an individual from the genomic data set. That is, the threshold risk criterion may indicate the probability that an individual may be identified from the genomic data set. For example, a threshold risk criterion of 0.05% would indicate an acceptable anonymization is achieved if the genomic data set provides a 0.05% risk of the person being re-identified. The threshold risk criterion may therefore be met if the calculated re-identification risk score is below the threshold risk criterion, and may not be met if the computed re-identification risk score is greater than the threshold re-identification.

In some embodiments, the re-identification risk score may be calculated as an applicable population, for example as indicated by Equation 1. In such embodiments, the threshold risk criterion may take the form of a raw number, such as a raw population size. That is, the threshold risk criterion may indicate a number of people within a population which the genomic data set could identify. The threshold re-identification risk score may therefore be met if the computed re-identification risk score (e.g. calculated applicable population) exceeds the threshold risk criterion.

If comparing 440 the re-identification risk score to the threshold risk criterion indicates that the re-identification risk score does not meet the threshold risk criterion, a phenotype informative SNP present in the genomic data may be selected 450 and masked 460. Masking the selected phenotype informative SNP may comprise deleting the data corresponding to the selected phenotype informative SNP in the genomic data set, replacing the data corresponding to the selected phenotype informative SNP with dummy data or null data, or otherwise obscuring the data corresponding to the selected phenotype informative SNP.

A phenotype informative SNP may be selected by identifying the phenotype informative SNP whose contribution term is the smallest when computing the applicable population. In other words, the phenotype informative SNP whose contribution is the highest when computing the re-identification risk score is identified, since the inverse of the applicable population may be used to calculate the re-identification risk score. The contribution term may be determined as described with reference to Figure 3, the contribution term corresponding to phenotypic trait contribution term **PT_cont** 370. In some embodiments, the contribution term for each phenotypic trait is stored, e.g. temporarily, with information identifying which phenotype informative SNP corresponds to said contribution term. the phenotype informative SNP whose corresponding risk term is the largest risk term **PT_r_max** 360 that contributes to the smallest contribution term **PT_cont** 370 may be selected in operation 450.

In some embodiments, one or more phenotype informative SNPs may have an associated priority indication. Data corresponding to phenotype informative SNPs with an associated priority indication may, in some embodiments, be preserved in the genomic data set such that they are not selected and masked in operations 450 and 460, respectively. For example, selecting 450 a phenotype informative SNP may comprise determining the smallest contribution term which is associated with a phenotype informative SNP (e.g. determining the contribution of the phenotype informative SNP which contributes the most to the re-identification risk score) without a priority indication. To illustrate this, consider the following example in which, for a particular phenotypic trait, the risk term **PT_r_1** 350-1 of **SNP_1** 320-1 in Figure 3 is the largest risk term for said phenotypic trait (e.g. **PT_r_1** 350-1 = **PT_r_max** 355), and the resulting contribution term **PT_cont** 370 is the smallest contribution term contributing to the re-identification risk score, which does not meet the threshold risk criterion. If **SNP_1** 320-1 has an associated priority indication, despite corresponding to the smallest contribution term, the data corresponding to **SNP_1** 320-1 may not be selected or masked. Instead, the next smallest contribution term and its associated phenotype informative SNP may be determined. If the phenotype informative SNP associated with the next smallest contribution does not have a priority indication, then said phenotype informative SNP may be selected in operation 450 and its corresponding data may be masked in operation 460.

Priority indications may be obtained by user input. In some embodiments, a user may have a particular interest in a subset of the phenotype informative SNPs and may wish to ensure that said subset is present in the anonymized data set. The user may, in such cases, input a list of SNPs of interest, either separately or as an additional field or flag in the genomic data set to be anonymized. In some embodiments, priority indications may be assigned automatically, based on a proximity to SNPs known to relate to a particular disease of interest. The proximity of an SNP to another SNP may be determined in any known method, for example using the genome pathways network described in patent application EP 3479272 A1, and in particular as described in page 4 line 28 to page 5 line 3, and page 6 line 18 to page 7 line 9. For example, SNPs within a predefined distance or proximity to an SNP of interest or to a SNP known to contribute to a particular disease may be prioritized by assigning a priority indication to such SNPs.

For example, a user may indicate a particular SNP of interest. The distance between each of the phenotype informative SNPs of the genomic data set and the indicated SNP may be determined, for example using the genomic pathways network. If, for a SNP, the distance between said SNP and the indicated SNP is below a threshold distance, then the SNP may be added to a subset of phenotype informative SNPs to which a priority indication may be applied.

Once a phenotype informative SNP has been masked in operation 460, the re-identification risk score may be calculated anew in operation 430, without the use of data corresponding to the masked phenotype informative SNP. That is, the phenotype informative SNP selected in operation 450 is effectively removed from the genomic data set. Subsequent calculations of the re-identification risk score may not include the use of data corresponding to such phenotype informative SNPs.

Phenotype informative SNPs may be removed from the genomic data set, e.g. by masking said phenotype informative SNPs, until the resulting re-identification risk score meets the threshold risk criterion. For example, if the threshold risk criterion denotes an acceptable risk level, the selecting and masking of phenotype informative SNPs and the recalculating of the re-identification risk score may repeat until the genomic data set is sufficiently anonymized.

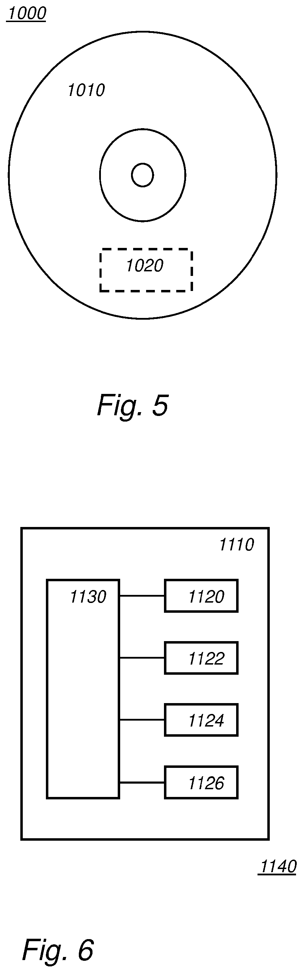

**Figure 5** schematically shows an example of a computer readable medium having a writable part comprising a computer program the computer program 1020 comprising instructions for causing a processor system to perform a method, such as the method of Figure 4. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said method of providing diagnosis support to a user.

**Figure 6** schematically shows a representation of a processor system 1140 according to an embodiment of the system 100 for anonymizing a genomic data set. The processor system comprises one or more integrated circuits 1110. The architecture of the one or more integrated circuits 1110 is schematically shown in Figure 6. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, which may be a bus. The processor system 1110 may be arranged for contact and/or contact-less communication, using an antenna and/or connectors, respectively.

For example, in an embodiment, processor system 1140, e.g., the system for anonymizing a genomic data set may comprise a processor circuit and a memory circuit, the processor being arranged to execute software stored in the memory circuit. For example, the processor circuit may be an Intel Core i7 processor, ARM Cortex-R8, etc. In an embodiment, the processor circuit may be ARM Cortex M0. The memory circuit may be an ROM circuit, or a non-volatile memory, e.g., a flash memory. The memory circuit may be a volatile memory, e.g., an SRAM memory. In the latter case, the system may comprise a non-volatile software interface, e.g., a hard drive, a network interface, etc., arranged for providing the software.

While the system 100 for anonymizing a genomic data set is shown as including one of each described component, the various components may be duplicated in various embodiments. For example, the processor 1120 may include multiple microprocessors that are configured to independently execute the methods described herein or are configured to perform steps or subroutines of the methods described herein such that the multiple processors cooperate to achieve the functionality described herein. Further, where the system 100 is implemented in a cloud computing system, the various hardware components may belong to separate physical systems. For example, the processor 1120 may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of" when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim. The scope of protection is defined by the claims.

**Claims**

1. A computer-implemented method for anonymizing a genomic data set, the genomic data set comprising a plurality of alleles arranged in a plurality of single nucleotide polymorphisms (SNPs), the plurality of SNPs comprising one or more phenotype informative SNPs, a phenotype informative SNP being an SNP relating to a phenotypic trait, the genomic data set corresponding to a genome of a person, the method comprising:

   - receiving (410) the genomic data set;
   - obtaining (420) a phenotypic probability for at least one phenotype informative SNP, a phenotypic probability being a probability of the phenotypic trait being expressed as a result of the at least one allele corresponding to the at least one phenotype informative SNP, and a proportion of a population which exhibits said phenotypic trait;
   - computing (430) a re-identification risk score based on the genomic data set, the re-identification risk score indicating a risk of re-identifying the person associated with the genomic data set from the genomic data set, the re-identification risk score being computed from the obtained phenotypic probability and the obtained proportion of the population which exhibits said phenotypic trait;
   - comparing (440) the re-identification risk score to a threshold risk criterion;
   - if the re-identification risk score does not meet the threshold risk criterion:

      - anonymizing the genomic data set by:

         - selecting (450) a phenotype informative SNP corresponding to the phenotypic traits considered in the calculation of the re-identification risk score, and
         - masking (460) the selected phenotype informative SNP; and

      - re-computing (430) the re-identification risk score;

   - if the re-identification risk score meets the threshold risk criterion:

      - outputting (470) the anonymized genomic data set.

2. The method of claim 1, wherein:

- comparing (440) the re-identification risk score to the threshold risk criterion,
- anonymizing (450, 460) the genomic data set, and
- re-computing (430) the re-identification risk score,

are repeated until the re-identification risk score meets the threshold risk criterion.

3. The method of claim 1 or claim 2, further comprising encrypting the anonymized genomic data set.

4. The method of any preceding claim, wherein computing (430) the re-identification risk score comprises:

- for each of at least one phenotypic trait:

- calculating a risk term of a phenotype informative SNP, the phenotype informative SNP relating to said phenotypic trait, the risk term being calculated from a genotypic frequency of the phenotype informative SNP and the phenotypic probability of said phenotypic trait associated with the at least one allele of the phenotype informative SNP, the genotypic frequency indicating a frequency of the at least one allele of the phenotype informative SNP in the population, and
- obtaining a proportion of the population which exhibits said phenotypic trait;

- computing the re-identification risk score from the calculated risk term of each of the at least one phenotypic trait and the proportion of the population obtained for each of the at least one phenotypic trait.

5. The method of claim 4, wherein computing the re-identification risk score comprises:

- for each of a plurality of phenotypic traits:

- obtaining a proportion of the population exhibiting said phenotypic trait;
- identifying at least one phenotype informative SNP relating to said phenotypic trait;
- calculating a risk term for each of the identified at least one phenotypic SNP;
- selecting the SNP having the largest risk term for said phenotypic trait; and
- determining a contribution term for said phenotypic trait from the obtained proportion of the population exhibiting said phenotypic trait and the risk term of the selected SNP; and

- determining an applicable population value from the contribution term for each of the plurality of phenotypic traits and the population; and
- computing the re-identification risk score based on the applicable population value.

6. The method of claim 4 or claim 5, wherein selecting (450) the phenotype informative SNP comprises selecting the SNP whose risk term is used to calculate a smallest contribution term, wherein the smallest contribution term of a phenotypic trait is defined as a proportion of the population exhibiting such phenotypic trait combined with a largest risk term (PT_r_max).

7. The method of any preceding claim, wherein the one or more phenotype informative SNPs comprises a subset of SNPs having a priority indication, and wherein selecting (450) the SNP comprises selecting a phenotype informative SNP without a priority indication, wherein the priority indication indicates SNPs to be preserved in the anonymized genomic data set.

8. The method of claim 7, wherein the subset of SNPs having the priority indication is identified by:

- for each SNP of the one or more phenotype informative SNPs:

- determine a distance using a genetic pathways network between the SNP and a prespecified SNP of interest;
- if the determined distance is within a threshold distance, adding said SNP to the subset of SNPs having the priority indication.

9. The method of any preceding claim, wherein masking the selected SNP comprises deleting a data entry in the genomic data set, the data entry representing the selected SNP.

10. The method of any preceding claim, further comprising outputting the re-identification risk score.

11. The method of any preceding claim, wherein computing the re-identification risk score comprises obtaining, from a database, statistical information regarding a dependency between multiple phenotypic traits, and applying a correction factor proportion of the population (PT_Pop) derived from the statistical information.

12. The method of any preceding claim, further comprising:

    - identifying at least one direct identifier, a direct identifier being a SNP which independently identifies the person; and
    - masking the identified at least one direct identifier in the genomic data set.

13. The method of any preceding claim, wherein the phenotypic trait comprises an exterior phenotypic trait.

14. A computer-readable medium comprising transitory or non-transitory data representing instructions which, when executed by a processor system, cause the processor system to perform the computer-implemented method according to any one of claims 1 to 13.

15. A system for anonymizing a genomic data set, the genomic data set comprising a plurality of alleles arranged in a plurality of single nucleotide polymorphisms (SNPs) the plurality of SNPs comprising one or more phenotype informative SNPs, a phenotype informative SNP being an SNP relating to a phenotypic trait, the genomic data set corresponding to a genome of a person, the system comprising:

    - an input/output subsystem (130) configured to:

        - receive the genomic data set;
        - obtain a phenotypic probability for at least one phenotype informative SNP, a phenotypic probability being a probability of the phenotypic trait being expressed as a result of the at least one allele corresponding to the at least one phenotype informative SNP, and a proportion of a population which exhibits said phenotypic trait;

    - a processor subsystem (110) configured to:

        - compute a re-identification risk score based on the genomic data set, the re-identification risk score indicating a risk of re-identifying the person associated with the genomic data set from the genomic data set, the re-identification risk score being computed from the obtained phenotypic probability and the obtained proportion of the population which exhibits said phenotypic trait;
        - compare the re-identification risk score to a threshold risk criterion;
        - if the re-identification risk score does not meet the threshold risk criterion:

            - anonymize the genomic data set by:

                - selecting a phenotype informative SNP corresponding to the phenotypic traits considered in the calculation of the re-identification risk score, and
                - masking the selected phenotype informative SNP; and

            - re-computing the re-identification risk score;

        - if the re-identification risk score meets the threshold risk criterion:

            - output, via the input/output subsystem (130) the anonymized genomic data set.

16. The system for anonymizing a genomic data set as claimed in claim 15, wherein the processor subsystem (110) is configured to select (450) the phenotype informative SNP by selecting the SNP whose risk term yields a smallest contribution term, wherein the smallest contribution term of a phenotypic trait is defined as a proportion of the population exhibiting such phenotypic trait combined with a largest risk term (PT_r_max).

17. The system for anonymizing a genomic data set as claimed in claim 15, wherein the processor subsystem (110) is configured to select the one or more phenotype informative SNPs as SNPs not having a priority indication, wherein the priority indication indicates SNPs to be preserved in the anonymized genomic data set.

**Patentansprüche**

1. Computerimplementiertes Verfahren zum Anonymisieren eines Genomdatensatzes, wobei der Genomdatensatz eine Vielzahl von Allelen umfasst, die in einer Vielzahl von Einzelnukleotid-Polymorphismen (SNPs) angeordnet sind, wobei die Vielzahl von SNPs einen oder mehrere phänotypinformative SNPs umfassen, wobei ein phänotypinformativer SNP ein SNP ist, der sich auf ein phänotypisches Merkmal bezieht, wobei der Genomdatensatz dem Genom einer Person entspricht, wobei das Verfahren Folgendes umfasst:

   - Empfangen (410) des Genomdatensatzes;
   - Erhalten (420) einer phänotypischen Wahrscheinlichkeit für mindestens einen phänotypinformativen SNP, wobei eine phänotypische Wahrscheinlichkeit eine Wahrscheinlichkeit ist, dass das phänotypische Merkmal infolgedessen ausgedrückt wird, dass das mindestens eine Allel dem mindestens einen phänotypinformativen SNP entspricht, und eines Anteils einer Population, der dieses phänotypische Merkmal aufweist;
   - Berechnen (430) eines Reidentifizierungsrisikowerts basierend auf dem Genomdatensatz, wobei der Reidentifizierungsrisikowert ein Risiko angibt, dass die Person reidentifiziert wird, die mit dem Genomdatensatz verknüpft ist, wobei der Reidentifizierungsrisikowert aus der erhaltenen phänotypischen Wahrscheinlichkeit und dem erhaltenen Anteil der Population, der das phänotypische Merkmal aufweist, berechnet wird;
   - Vergleichen (440) des Reidentifizierungsrisikowerts mit einem Schwellenwertrisikokriterium;
   - falls der Reidentifizierungsrisikowert das Schwellenwertrisikokriterium nicht erfüllt:

      - Anonymisieren des Genomdatensatzes durch:

         - Auswählen (450) eines phänotypinformativen SNP, der den bei der Berechnung des Reidentifizierungsrisikowerts berücksichtigten phänotypischen Merkmalen entspricht, und
         - Maskieren (460) des ausgewählten phänotypinformativen SNP; und

      - Neuberechnen (430) des Reidentifizierungsrisikowerts;

   - wenn der Reidentifizierungsrisikowert das Schwellenwertrisikokriterium erfüllt:

      - Ausgeben (470) des anonymisierten Genomdatensatzes.

2. Verfahren nach Anspruch 1, wobei:

   - Vergleichen (440) des Reidentifizierungsrisikowerts mit dem Schwellenwertrisikokriterium,
   - Anonymisieren (450, 460) des Genomdatensatzes und
   - Neuberechnen (430) des Reidentifizierungsrisikowerts wiederholt werden, bis der Reidentifizierungsrisikowert das Schwellenwertkriterium erfüllt.

3. Verfahren nach Anspruch 1 oder Anspruch 2, das ferner Verschlüsseln des anonymisierten Genomdatensatzes umfasst.

4. Verfahren nach einem vorstehenden Anspruch, wobei Berechnen (430) des Reidentifizierungsrisikowerts Folgendes umfasst:

   - für jedes von mindestens einem phänotypischen Merkmal:

      - Berechnen eines Risikoterms eines phänotypinformativen SNP, wobei sich der phänotypinformative SNP auf das genannte phänotypische Merkmal bezieht, wobei der Risikoterm aus einer genotypischen Frequenz des phänotypinformativen SNP und der phänotypischen Wahrscheinlichkeit des phänotypischen Merkmals, das mit dem mindestens einen Allel des phänotypinformativen SNP verknüpft ist, berechnet wird, wobei die genotypische Frequenz eine Frequenz des mindestens einen Allels des phänotypinformativen SNP in der Population angibt, und

- Erhalten eines Anteils der Population, der das phänotypische Merkmal aufweist;
- Berechnen des Reidentifizierungsrisikowerts aus dem berechneten Risikoterm jedes des mindestens einen phänotypischen Merkmals und des Anteils der Population, der für jedes des mindestens einen phänotypischen Merkmals erhalten wurde.

**5.** Verfahren nach Anspruch 4, wobei Berechnen des Reidentifizierungsrisikowerts Folgendes umfasst:

- für jedes einer Vielzahl von phänotypischen Merkmalen:

- Erhalten eines Anteils der Population, der das phänotypische Merkmal aufweist;
- Identifizieren mindestens eines phänotypinformativen SNP, der sich auf das phänotypische Merkmal bezieht;
- Berechnen eines Risikoterms für jeden des identifizierten mindestens einen phänotypischen SNP;
- Auswählen des SNP, der den größten Risikoterm für das genannte phänotypische Merkmal aufweist; und
- Bestimmen eines Beitragsterms für das phänotypische Merkmal aus dem erhaltenen Anteil der Population, der das phänotypische Merkmal aufweist, und dem Risikoterm des ausgewählten SNP; und

- Bestimmen eines anwendbaren Populationswerts aus dem Beitragsterm für jedes der Vielzahl von phänotypischen Merkmalen und der Population; und
- Berechnen des Reidentifizierungsrisikowerts basierend auf dem entsprechenden Populationswert.

**6.** Verfahren nach Anspruch 4 oder Anspruch 5, wobei Auswählen (450) des phänotypinformativen SNP Auswählen des SNP umfasst, dessen Risikoterm verwendet wird, um einen kleinsten Beitragsterm zu berechnen, wobei der kleinste Beitragsterm eines phänotypischen Merkmals als ein Anteil der Population, der dieses phänotypische Merkmal aufweist, kombiniert mit einem größten Risikoterm (PT_r_max) definiert ist.

**7.** Verfahren nach einem vorstehenden Anspruch, wobei der eine oder die mehreren phänotypinformativen SNPs eine Teilmenge von SNPs umfassen, die eine Prioritätsangabe aufweisen, und wobei Auswählen (450) des SNP Auswählen eines phänotypinformativen SNP ohne eine Prioritätsangabe umfasst, wobei die Prioritätsangabe SNPs angibt, die im anonymisierten Genomdatensatz erhalten bleiben sollen.

**8.** Verfahren nach Anspruch 7, wobei die Teilmenge der SNPs, die die Prioritätsangabe aufweisen, wie folgt identifiziert wird:

- für jeden SNP des einen oder der mehreren phänotypinformativen SNPs:

- Bestimmen, unter Verwendung eines genetischen Pfadnetzwerks, eines Abstands zwischen dem SNP und einem vorab festgelegten SNP von Interesse;
- wenn der bestimmte Abstand innerhalb eines Schwellenwertabstands liegt, Hinzufügen des SNP zu der Teilmenge von SNPs, die die Prioritätsangabe aufweisen.

**9.** Verfahren nach einem vorstehenden Anspruch, wobei Maskieren des ausgewählten SNP Löschen eines Dateneintrags im Genomdatensatz umfasst, wobei der Dateneintrag den ausgewählten SNP darstellt.

**10.** Verfahren nach einem vorstehenden Anspruch, das ferner Ausgeben des Reidentifizierungsrisikowerts umfasst.

**11.** Verfahren nach einem vorstehenden Anspruch, wobei Berechnen des Reidentifizierungsrisikowerts Erhalten, von einer Datenbank, statistischer Informationen bezüglich einer Abhängigkeit zwischen mehreren phänotypischen Merkmalen und Anwenden eines Korrekturfaktoranteils der Population (PT Pop), der aus den statistischen Informationen abgeleitet wird, umfasst.

**12.** Verfahren nach einem vorstehenden Anspruch, das ferner Folgendes umfasst:

- Identifizieren mindestens einer direkten Kennung, wobei eine direkte Kennung ein SNP ist, die die Person unabhängig identifiziert; und
- Maskieren der identifizierten mindestens einen direkten Kennung im Genomdatensatz.

**13.** Verfahren nach einem vorstehenden Anspruch, wobei das phänotypische Merkmal ein äußeres phänotypisches Merkmal umfasst.

14. Computerlesbares Medium, das flüchtige oder nichtflüchtige Daten umfasst, welche Anweisungen darstellen, die, wenn sie von einem Prozessorsystem ausgeführt werden, das Prozessorsystem veranlassen, das computerimplementierte Verfahren nach einem der Ansprüche 1 bis 13 durchzuführen.

15. System zum Anonymisieren eines Genomdatensatzes, wobei der Genomdatensatz eine Vielzahl von Allelen umfasst, die in einer Vielzahl von Einzelnukleotid-Polymorphismen (SNPs) angeordnet sind, wobei die Vielzahl von SNPs einen oder mehrere phänotypinformative SNPs umfassen, wobei ein phänotypinformativer SNP ein SNP ist, der sich auf ein phänotypisches Merkmal bezieht, wobei der Genomdatensatz dem Genom einer Person entspricht, wobei das System Folgendes umfasst:

- ein Eingabe/Ausgabe-Subsystem (130), das für Folgendes konfiguriert ist:

- Empfangen des Genomdatensatzes;
- Erhalten einer phänotypischen Wahrscheinlichkeit für mindestens einen phänotypinformativen SNP, wobei die phänotypische Wahrscheinlichkeit eine Wahrscheinlichkeit ist, dass das phänotypische Merkmal infolgedessen ausgedrückt wird, dass das mindestens eine Allel dem mindestens einen phänotypinformativen SNP entspricht, und eines Anteils einer Population, der dieses phänotypische Merkmal aufweist;

- ein Prozessorsubsystem (110), das für Folgendes konfiguriert ist:

- Berechnen eines Reidentifizierungsrisikowerts basierend auf dem Genomdatensatz, wobei der Reidentifizierungsrisikowert ein Risiko angibt, dass die Person reidentifiziert wird, die mit dem Genomdatensatz verknüpft ist, wobei der Reidentifizierungsrisikowert aus der erhaltenen phänotypischen Wahrscheinlichkeit und dem erhaltenen Anteil der Population, der das phänotypische Merkmal aufweist, berechnet wird;
- Vergleichen des Reidentifizierungsrisikowerts mit einem Schwellenwertrisikokriterium;
- falls der Reidentifizierungsrisikowert das Schwellenwertrisikokriterium nicht erfüllt:

- Anonymisieren des Genomdatensatzes durch:

- Auswählen eines phänotypinformativen SNP, der den bei der Berechnung des Reidentifizierungsrisikowerts berücksichtigten phänotypischen Merkmalen entspricht, und
- Maskieren des ausgewählten phänotypinformativen SNP; und

- Neuberechnen des Reidentifizierungsrisikowerts;

- wenn der Reidentifizierungsrisikowert das Schwellenwertrisikokriterium erfüllt:

- Ausgeben, über das Eingabe/Ausgabe-Subsystem (130), des anonymisierten Genomdatensatzes.

16. System zum Anonymisieren eines Genomdatensatzes nach Anspruch 15, wobei das Prozessorsubsystem (110) konfiguriert ist zum Auswählen (450) des phänotypinformativen SNP durch Auswählen des SNP, dessen Risikoterm einen kleinsten Beitragsterm ergibt, wobei der kleinste Beitragsterm eines phänotypischen Merkmals als ein Anteil der Population, der dieses phänotypische Merkmal aufweist, kombiniert mit einem größten Risikoterm (PT_r_max) definiert ist.

17. System zum Anonymisieren eines Genomdatensatzes nach Anspruch 15, wobei das Prozessorsubsystem (110) konfiguriert ist zum Auswählen des einen oder der mehreren phänotypinformativen SNPs als SNPs, die keine Prioritätsangabe aufweisen, wobei die Prioritätsangabe SNPs angibt, die im anonymisierten Genomdatensatz erhalten bleiben sollen.

**Revendications**

1. Procédé informatique d'anonymisation d'un ensemble de données génomiques, l'ensemble de données génomiques comprenant une pluralité d'allèles agencés en une pluralité de polymorphismes mononucléotidiques (SNP), la pluralité de SNP comprenant un ou plusieurs SNP informatifs de phénotype, un SNP informatif de phénotype étant un SNP relatif à un caractère phénotypique, l'ensemble de données génomiques correspondant au génome d'une personne, le procédé comprenant :

- la réception (410) de l'ensemble de données génomiques ;
- l'obtention (420) d'une probabilité phénotypique pour au moins un SNP informatif de phénotype, une probabilité phénotypique étant une probabilité que le caractère phénotypique soit exprimé à la suite du au moins un allèle correspondant à le au moins un SNP informatif de phénotype, et une proportion d'une population qui présente ledit caractère phénotypique ;
- le calcul (430) d'un score de risque de ré-identification basé sur l'ensemble de données génomiques, le score de risque de ré-identification indiquant un risque de ré-identification de la personne associée à l'ensemble de données génomiques à partir de l'ensemble de données génomiques, le score de risque de ré-identification étant calculé à partir de la probabilité phénotypique obtenue et de la proportion obtenue de la population qui présente ledit caractère phénotypique ;
- la comparaison (440) du score de risque de ré-identification à un critère de risque seuil ;
- si le score de risque de ré-identification ne satisfait pas le critère de risque seuil :

  - l'anonymisation de l'ensemble de données génomiques en :

    - sélectionnant (450) un SNP informatif de phénotype correspondant aux caractères phénotypiques considérés dans le calcul du score de risque de ré-identification, et
    - masquant (460) le SNP informatif de phénotype sélectionné ; et

  - recalculant (430) le score de risque de ré-identification ;

- si le score de risque de ré-identification atteint le critère de risque seuil :

  - l'affichage (470) de l'ensemble de données génomiques anonymisées.

2. Procédé selon la revendication 1, dans lequel :

  - la comparaison (440) du score de risque de ré-identification au critère de risque seuil,
  - l'anonymisation (450, 460) de l'ensemble de données génomiques, et
  - le fait de recalculer (430) le score de risque de ré-identification, sont répétées jusqu'à ce que le score de risque de ré-identification satisfasse le critère de risque seuil.

3. Procédé selon la revendication 1 ou la revendication 2, comprenant en outre un chiffrement de l'ensemble de données génomiques anonymisées.

4. Procédé selon une quelconque revendication précédente, dans lequel le calcul (430) du score de risque de ré-identification comprend :

  - pour chacun d'au moins un caractère phénotypique :

    - le calcul d'un terme de risque d'un SNP informatif de phénotype, le SNP informatif de phénotype relatif audit caractère phénotypique, le terme de risque étant calculé à partir d'une fréquence génotypique du SNP informatif de phénotype et de la probabilité phénotypique dudit caractère phénotypique associée à l'au moins un allèle du SNP informatif de phénotype, la fréquence génotypique indiquant une fréquence de l'au moins un allèle du SNP informatif de phénotype dans la population, et

  - l'obtention d'une proportion de la population qui présente ledit caractère phénotypique ;
  - le calcul du score de risque de ré-identification à partir du terme de risque calculé de chacun des au moins un caractère phénotypique et de la proportion de la population obtenue pour chacun des au moins un caractère phénotypique.

5. Procédé selon la revendication 4, dans lequel le calcul du score de risque de ré-identification comprend :

  - pour chacun d'une pluralité de caractères phénotypiques :

- l'obtention d'une proportion de la population présentant ledit caractère phénotypique ;
- l'identification d'au moins un SNP informatif de phénotype relatif audit caractère phénotypique ;
- le calcul d'un terme de risque pour chacun des au moins un SNP phénotypique identifié ;
- la sélection du SNP présentant le risque le plus élevé pour ledit caractère phénotypique ; et
- la détermination d'un terme de contribution pour ledit caractère phénotypique à partir de la proportion obtenue de la population présentant ledit caractère phénotypique et du terme de risque du SNP sélectionné ; et

- la détermination d'une valeur de population applicable à partir du terme de contribution pour chacun des caractères phénotypiques et la population ; et
- le calcul du score de risque de ré-identification basé sur la valeur de population applicable.

6. Procédé selon la revendication 4 ou 5, dans lequel la sélection (450) du SNP informatif de phénotype comprend la sélection du SNP dont le terme de risque est utilisé pour calculer un terme de contribution minimal, dans lequel le terme de contribution minimal d'un caractère phénotypique est défini comme une proportion de la population présentant un tel caractère phénotypique combiné à un terme de risque maximal (PT_r_max).

7. Procédé selon une quelconque revendication précédente, dans lequel les un ou plusieurs SNP informatifs de phénotype comprennent un sous-ensemble de SNP présentant une indication de priorité, et dans lequel la sélection (450) du SNP comprend la sélection d'un SNP informatif de phénotype sans indication de priorité, dans lequel l'indication de priorité indique les SNP à préserver dans l'ensemble de données génomiques anonymisées.

8. Procédé selon la revendication 7, dans lequel le sous-ensemble de SNP présentant l'indication de priorité est identifié par :

- pour chaque SNP des un ou plusieurs SNP informatifs de phénotype :

- la détermination d'une distance à l'aide d'un réseau de voies génétiques entre le SNP et un SNP d'intérêt prédéfini ;
- si la distance déterminée se situe dans une distance seuil, l'ajout dudit SNP au sous-ensemble de SNP présentant l'indication de priorité.

9. Procédé selon une quelconque revendication précédente, dans lequel le masquage du SNP sélectionné comprend une suppression d'une entrée de données dans l'ensemble de données génomiques, l'entrée de données représentant le SNP sélectionné.

10. Procédé selon une quelconque revendication précédente, comprenant en outre l'émission du score de risque de ré-identification.

11. Procédé selon une quelconque revendication précédente, dans lequel le calcul du score de risque de ré-identification comprend l'obtention, à partir d'une base de données, d'informations statistiques concernant une dépendance entre plusieurs caractères phénotypiques, et l'application d'un facteur de correction proportion de la population (PT Pop) dérivé des informations statistiques.

12. Procédé selon une quelconque revendication précédente, comprenant en outre :

- l'identification d'au moins un identifiant direct, un identifiant direct étant un SNP qui identifie indépendamment la personne ; et
- le masquage d'au moins un identifiant direct identifié dans l'ensemble de données génomiques.

13. Procédé selon une quelconque revendication précédente, dans lequel le caractère phénotypique comprend un caractère phénotypique extérieur.

14. Support lisible par ordinateur comprenant des données transitoires ou non transitoires représentant des instructions qui, lorsqu'elles sont exécutées par un système de processeur, amènent le processeur à exécuter le procédé informatique selon l'une quelconque des revendications 1 à 13.

15. Système d'anonymisation d'un ensemble de données génomiques, l'ensemble de données génomiques comprenant une pluralité d'allèles agencés en une pluralité de polymorphismes mononucléotidiques (SNP), la pluralité de SNP comprenant un ou plusieurs SNP informatifs de phénotype, un SNP informatif de phénotype étant un SNP relatif à un caractère phénotypique, l'ensemble de données génomiques correspondant au génome d'une personne, le système comprenant :

    - un sous-système d'entrée/sortie (130) configuré pour :

        - recevoir l'ensemble de données génomiques ;
        - obtenir une probabilité phénotypique pour au moins un SNP informatif de phénotype, une probabilité phénotypique étant une probabilité que le caractère phénotypique soit exprimé à la suite du au moins un allèle correspondant à le au moins un SNP informatif de phénotype, et une proportion d'une population qui présente ledit caractère phénotypique ;

    - un sous-système de processeur (110) configuré pour :

        - calculer un score de risque de ré-identification basé sur l'ensemble de données génomiques, le score de risque de ré-identification indiquant un risque de ré-identification de la personne associée à l'ensemble de données génomiques à partir de l'ensemble de données génomiques, le score de risque de ré-identification étant calculé à partir de la probabilité phénotypique obtenue et de la proportion obtenue de la population qui présente ledit caractère phénotypique ;
        - comparer le score de risque de ré-identification à un critère de risque seuil ;
        - si le score de risque de ré-identification ne satisfait pas le critère de risque seuil :

            - anonymiser l'ensemble de données génomiques en :

                - sélectionnant un SNP informatif de phénotype correspondant aux caractères phénotypiques considérés dans le calcul du score de risque de ré-identification, et
                - masquant le SNP informatif de phénotype sélectionné ; et

            - recalculant le score de risque de ré-identification ;

        - si le score de risque de ré-identification atteint le critère de risque seuil :

            - émettre, via le sous-système d'entrée/sortie (130) l'ensemble de données génomiques anonymisées.

**16.** Système d'anonymisation d'un ensemble de données génomiques selon la revendication 15, dans lequel le sous-système de processeur (110) est configuré pour sélectionner (450) le SNP informatif de phénotype en sélectionnant le SNP dont le terme de risque produit le terme de contribution le plus faible, dans lequel le terme de contribution le plus faible d'un caractère phénotypique est défini comme la proportion de la population présentant un tel caractère phénotypique combiné au terme de risque le plus élevé (PT_r_max).

**17.** Système d'anonymisation d'un ensemble de données génomiques selon la revendication 15, dans lequel le sous-système de processeur (110) est configuré pour sélectionner les un ou plusieurs SNP informatifs de phénotype comme SNP ne présentant pas d'indication de priorité, dans lequel l'indication de priorité indique les SNP à préserver dans l'ensemble de données génomiques anonymisées.

*Fig. 1*

| SNP | Genotype | Allele frequency 1 | Allele frequency 2 | Genotype frequency | Phenotype | Related to PCa |
|---|---|---|---|---|---|---|
| SNP_E1* | AA | 80% | 80% | 64% | 40% blue eyes | Yes |
| SNP_E2 | AG | 90% | 5% | 4.5% | 80% blue eyes | |
| SNP_E3 | GT | 50% | 40% | 20% | 95% blue eyes | Yes |
| SNP_E4 | CC | 90% | 90% | 81% | 50% blue eyes | |
| SNP_E5 | CT | 70% | 25% | 17.5% | 70% blue eyes | |
| SNP_H1 | GG | 90% | 90% | 81% | 70% brown hair | Yes |
| SNP_H2 | CC | 80% | 80% | 64% | 85% brown hair | Yes |
| SNP_H3 | TA | 70% | 20% | 14% | 90% brown hair | |
| SNP_S1* | AA | 80% | 80% | 64% | 55% light skin | Yes |
| SNP_S2 | GC | 75% | 20% | 15% | 35% light skin | |
| SNP_S3 | GG | 80% | 80% | 64% | 80% light skin | |
| SNP_S4 | AC | 50% | 40% | 20% | 70% light skin | Yes |

*Fig. 2*

EP 4 238 099 B1

*Fig. 3*

EP 4 238 099 B1

*Fig. 4*

*1000*

## Fig. 5

## Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200035332 A1

- EP 3479272 A1

**Non-patent literature cited in the description**

- **KALE GULCE et al.** A utility maximizing and privacy preserving approach for protecting kinship in genomic databases. *Bioinformatics*, 2018, vol. 34 (2)

- **HUMBERT MATHIAS et al.** De-anonymizing Genomic Databases Using Phenotypic Traits. *Proceedings on Privacy Enhancing Technologies*, 01 June 2015, vol. 2015 (2)